# FASCICULE DE BREVET EUROPEEN

(11) **EP 4 027 862 B1**
(45) Date de publication et mention de la délivrance du brevet: **19.03.2025**
(21) Numéro de dépôt: 20781047.4
(22) Date de dépôt: 11.09.2020
(51) Int. Cl.: A61B 3/10

(54) **PROCÉDÉ D'ÉVALUATION DE LA STABILITÉ D'UN FILM LACRYMAL**
VERFAHREN ZUR BEURTEILUNG DER STABILITÄT EINES TRÄNENFILMS
METHOD FOR EVALUATING THE STABILITY OF A TEAR FILM

(30) Priorité: 13.09.2019 FR 1910140
(43) Date de publication de la demande: 20.07.2022
(73) Titulaire: E-Swin Developpement, 78550 Houdan (FR)
(72) Inventeur: BROTTIER, Yves-Vincent, 78113 ADAINVILLE (FR); OBIN, Arnaud, 78660 PARAY-DOUAVILLE (FR); PERRIN, Nelson, 78730 SAINTE-MESME (FR)
(74) Mandataire: Plasseraud IP
(86) Numéro de dépôt international: PCT/FR2020/051579
(87) Numéro de publication internationale: WO 2021/048510

(56) Documents cités:
- WO-A1-2015/073986
- WO-A1-2015/073986
- WO-A1-2018/156022
- WO-A1-2018/156022

## Description

### Domaine technique

L'invention relève du domaine de la détection du syndrome de sécheresse oculaire.

### Technique antérieure

Les tests d'évaluation du syndrome de sécheresse oculaire font traditionnellement appel à un test par fluorescence. Le praticien instille dans l'œil du patient une goutte de fluorescéine et le phénomène de fluorescence est observé à l'aide d'une lampe à fente. Le praticien détermine le temps entre le clignement de paupière et l'apparition de la première zone non fluorescente. Ce temps est la mesure du temps de rupture du film oculaire ou Breakup Time en anglais ou BUT. Cette méthode conduit à mesurer une première rupture, qu'elle soit due à un défaut local tel qu'une poussière ou un réel problème de sécheresse oculaire. Les temps mesurés sont de l'ordre de 6 à 10 secondes.

Une autre méthode d'évaluation, dénommée « Non-Invasive Breakup Time en anglais» ou NIBUT est un procédé non invasif de mesure du temps de rupture. Les tests de cette méthode utilisent notamment la réflexion sur la cornée d'une mire pourvue d'un motif constitué de cercles concentriques et de lignes radiales

Lorsqu'une rupture du film apparaît, le motif réfléchi présente des déformations des lignes et/ou des cercles ou des zones manquantes.

Les images du motif réfléchi sont acquises en séquences par une caméra et traitées par un système de traitement informatisé qui analyse l'évolution du motif dans le temps pour détecter les déformations du motif et en déduire le temps d'apparition des zones de rupture du film.

WO 2018/156022 révèle un procédé de mesure de l'état du film lacrymal utilisant un dispositif comprenant:
- un stimulateur multicolore 100 comprenant une surface hémisphérique 120 sur laquelle sont montés des DELs aptes à projeter des rayons lumineux sur la cornée en forme de grille rectangulaire,
- une caméra pour enregistrer une image de l'éclairage de la cornée,
- un ordinateur pour traiter les images enregistrées et déterminer une distorsion locale de chaque point d'image et l'intervalle entre clignement de l'œil et rupture du film lacrymal.

### Problème technique

Les dispositifs actuellement disponibles utilisant des motifs circulaires et de lignes radiales occasionnent des problèmes de plusieurs ordres :
compte tenu de la forme des mires, et de la couverture de la cornée par secteurs de disque, la résolution spatiale est intrinsèquement moins bonne sur les bords du champ qu'au centre.

Par ailleurs au centre du champ, là où les lignes radiales convergent, il est difficile de déterminer la déformation d'un motif.

L'augmentation de la résolution spatiale nécessiterait de resserrer le motif de la mire. Or, les algorithmes de détection de déformations de cercles qui requièrent de trouver les cercles les plus probables dans l'image, d'évaluer si un point de l'image appartient à un cercle probable et de calculer la distance d'un point donné au cercle auquel il est censé appartenir sont complexes et le temps de traitement et de calcul pour détecter les zones de rupture à partir de l'image est important.

Il est donc clair que : d'une part les dispositifs présentant le motif classique de cercles concentriques et lignes radiales souffrent d'un défaut d'homogénéité de la résolution spatiale, d'autre part augmenter la résolution en bord de champ est pénalisant sur les temps de calcul.

Du fait de la faible résolution de ce procédé, la mesure peut donner des valeurs d'apparition de ruptures imprécises et surévaluées.

Il faut noter notamment que les mesures obtenues par la méthode par fluorescence et la méthode non invasive ne sont généralement pas bien corrélées : un facteur 2 entre les mesures BUT et NIBUT est couramment observé, la mesure par fluorescence affichant un temps plus court. Ceci amène à penser à un manque de sensibilité ou de résolution des appareils NIBUT.

De même les temps affichés en NIBUT sur différents appareils disponibles ne sont pas non plus bien corrélés.

Dans le diagnostic du syndrome de l'œil sec, les mesures obtenues par la mesure du break up time obtenues avec de la fluorescéine ou les mesures obtenues par une méthode de NIBUT ne sont pas corrélées avec la souffrance exprimée par le patient.

Les méthodes précédentes ne sont pas révélatrices de l'état global du film lacrymal ce qui est préjudiciable au praticien qui a besoin de plus de précision et de répétabilité des mesures pour effectuer son diagnostic.

### Exposé de l'invention

L'invention vient améliorer la situation et propose un procédé de mesure de stabilité d'un film lacrymal sur la cornée d'un patient, à partir de la détection de modifications de ce film et utilisant un dispositif comportant un motif constitué à partir de lignes claires et sombres horizontales ou verticales, et d'observer la réflexion de ce motif sur la cornée.

Pour ce faire, dans le cadre de la présente demande on va rechercher les micro-mouvements au niveau du film lacrymal en analysant les images des lignes de la mire.

On appelle micro-mouvement une variation locale de l'épaisseur globale du film lacrymal. Cette variation induit des pentes sur la surface du film lacrymal, qui seront matérialisées par une modification localisée de l'image des lignes de la mire et notamment des modifications de la largeur de l'image de ces lignes ou des déformations locales.

Plus précisément, la présente demande propose un procédé de mesure de stabilité d'un film lacrymal d'un patient au moyen d'un dispositif comportant une plaque translucide rétroéclairée munie d'une mire se positionnant devant au moins un œil d'un patient, au moins une caméra photographique numérique reliée à un système de calcul pourvu de moyens de traitement et d'analyse d'images, un objectif de la caméra pointant vers l'œil du patient afin de photographier une réflexion du motif de la mire sur l'œil du patient, caractérisé en ce que, la mire étant pourvue d'un motif constitué d'une succession de lignes claires et sombres alternées se réfléchissant sur l'œil du patient, les moyens de traitement et d'analyse d'images sont configurés pour détecter des déformations desdites lignes claires ou sombres du motif de la mire réfléchi sur l'œil du patient et identifier, par une comparaison de la position de points images du bord des lignes par rapport à un bord des lignes estimé, des micro-mouvements du film lacrymal révélés par ces déformations, ledit procédé comportant, à partir d'une origine des temps t0 constituée par un clignement de paupière, une succession de prises d'images, une succession de détections de zones de micro mouvements dans les images et une succession de calculs et mémorisation de données de positions et de nombre de zones de micro-mouvements du film lacrymal dans chaque image au moyen des moyens de traitement et d'analyse d'images et le calcul, à partir de la succession de calculs de positions et de nombre de zones de micro-mouvements du film lacrymal, d'une mesure de l'amplitude moyenne des zones de micro-mouvements en fonction du temps.

La mesure effectuée a l'avantage de donner une image des variations d'épaisseur du film oculaire, film trop fin ou rompu localement au long d'une séquence de mesure.

Les caractéristiques exposées dans les paragraphes suivants peuvent, optionnellement, être mises en œuvre. Elles peuvent être mises en œuvre indépendamment les unes des autres ou en combinaison les unes avec les autres :
Le procédé peut comporter une prise d'image (200) toutes les 0,1 à 0,5 secondes et préférablement toutes les 0,3 secondes.

Ladite succession de prises d'images se termine avantageusement avec le premier arrivé de l'un des évènements : fin d'une temporisation ou détection d'un clignement de paupière suivant.

Le procédé peut comporter un suivi de l'œil ou des yeux du patient au moyen d'un procédé de suivi d'iris en sorte de repositionner les zones de micro-mouvements du film lacrymal détectées et mémorisées par rapport à l'œil analysé.

Les bords des lignes estimés peuvent être calculés par régression polynomiale.

Le procédé peut comporter la détermination d'une population de points représentatifs des zones de micro-mouvements observées sur l'image des lignes par calcul, le long des lignes, de la valeur absolue de la distance au polynôme selon l'axe perpendiculaire à la direction générale des lignes des pixels P1 à Pn des bords de lignes de l'image qui sont écartés du polynôme d'une distance dP en pixels supérieure à un seuil et mémorisation pour les points représentatifs P1 à Pn des distances dP1 à dPn.

Le procédé peut comporter pour chaque image, sur ladite population de points représentatifs, un calcul pour chaque pixel P1 à Pn de la surface équivalente Sp = dP x largeur pixel x hauteur pixel et un calcul de la somme ΣSp des surfaces équivalents SP1 à SPn des points image à leur polynôme sur toute l'image et un calcul d'une somme normalisée NΣSp desdites distances au moyen de la division de la somme ΣSp par la longueur totale des lignes trouvées sur l'image.

Le procédé peut comporter le calcul d'un score global constitué par la somme A des sommes normalisées NΣSp sur une série d'images à partir de l'origine des temps t0 jusqu'à un instant t donné.

La durée des mesures peut être choisie en fonction d'une population de patients, une durée de l'ordre de 6 secondes apparaissant suffisante pour détecter des problèmes de sécheresse oculaire tout en évitant d'atteindre un risque de clignement d'œil.

Le procédé peut comporter une mémorisation des positions des zones de micro-mouvements et la génération d'une cartographie des localisations des micro-mouvements image par image.

Le procédé peut comporter un calcul et la mémorisation du temps d'apparition des points de micro-mouvement du film lacrymal et/ou de la cinétique d'apparition de points de micro-mouvements du film lacrymal.

Le procédé peut comporter une mémorisation dans une base de données de toute ou partie des données calculées en sorte de réaliser un suivi du patient et une comparaison desdites données sur plusieurs examens.

Selon un autre aspect, il est proposé un programme informatique comportant des instructions pour la mise en œuvre de tout ou partie d'un procédé tel que défini dans les présentes lorsque ce programme est exécuté par un processeur.

Selon un autre aspect de l'invention, il est proposé un support d'enregistrement non transitoire, lisible par un ordinateur, sur lequel est enregistré un tel programme.

L'invention et les variantes de celle-ci peuvent permettre, de manière générale, de proposer une méthode de mesure de l'évolution des irrégularités d'un film lacrymal sur une durée d'examen définie offrant une précision accrue par rapport aux méthodes existantes de détection de ruptures d'un tel film lacrymal.

Une telle solution qui se base sur une mesure des déformations du film et leur évolution dans le temps permet de résoudre les problèmes posés par les solutions connues en offrant une grande répétabilité des mesures.

### Brève description des dessins

D'autres caractéristiques, détails et avantages de l'invention apparaîtront à la lecture de la description détaillée ci-après, et à l'analyse des dessins annexés, sur lesquels :
[Fig. 1] est une vue à plat de face d'une mire pourvue d'un motif de l'invention ;
[Fig. 2] est une vue schématique d'un dispositif de la présente demande;
[Fig. 3] montre un premier exemple de support de mesures utilisable dans le cadre de la présente demande ;
[Fig. 4] représente une image d'un œil d'un patient suite à une première étape de traitement ;
[Fig. 5A], [Fig. 5B], [Fig. 5C], [Fig. 5D], [Fig. 5E] représentent diverses étapes de traitement de l'image de l'œil de la figure 4;
[Fig. 6A], [Fig. 6B] représentent des détails de la figure 5E;
[Fig. 7] représente une image de l'œil de la figure 4 après analyse d'image ;
[Fig. 8] représente une image d'un œil d'un patient qui regarde vers une caméra ;
[Fig. 9A], [Fig. 9B], [Fig. 9C] représentent des étapes de détermination de la position de l'iris de l'œil de la figure 8 selon un aspect de la demande;
[Fig. 10] représente une image d'un œil d'un patient dont le regard s'écarte d'une caméra ;
[Fig. 11A], [Fig. 11B], [Fig. 11C] représentent des étapes de détermination de la position de l'iris de l'œil de la figure 10 selon un aspect de la demande;
[Fig. 12] schématise un procédé de détection de points décalés selon un aspect de la demande;
[Fig. 13] schématise un procédé de repositionnement de déformations de lignes d'image d'une mire ;
[Fig. 14] Schématise des premières étapes de procédé de la demande ;
[Fig. 15] schématise des étapes ultérieures de procédé de la demande ;
[Fig. 16] représente un premier graphique avec courbes de valeurs lissées pour plusieurs patients ;
[Fig. 17] représente un second graphique avec courbes de valeurs cumulées pour plusieurs patients.

### Description des modes de réalisation

Les dessins et la description ci-après décrivent un ou plusieurs exemples de réalisation qui pourront donc non seulement servir à mieux faire comprendre les objets de la présente demande, mais aussi contribuer à sa définition, le cas échéant.

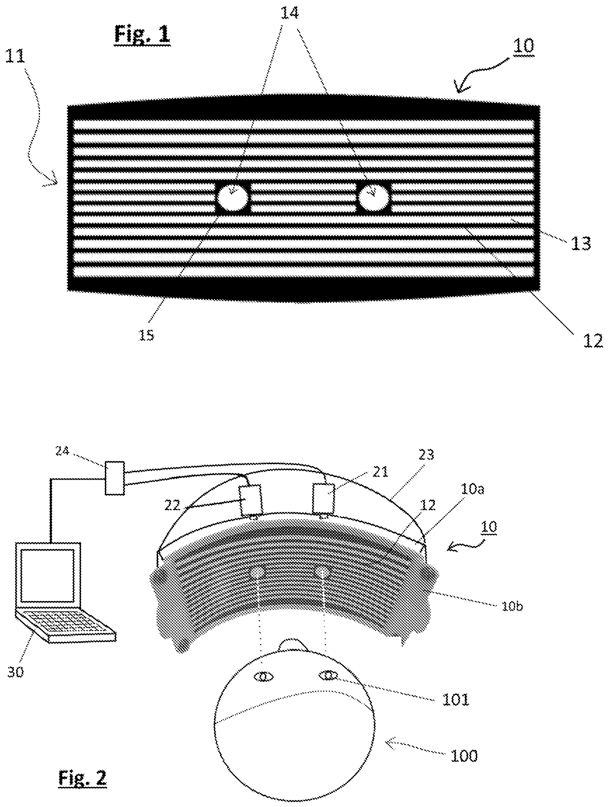

Le procédé selon la présente demande utilise un dispositif de mesure comportant une mire 10 représentée en figure 1 et réalisée à partir d'un film polymère transparent comportant un motif 11 réalisé avec une alternance de lignes 12, 13 droites et parallèles. Le motif comporte des lignes opaques 12, par exemple des lignes noires séparées par des lignes transparentes 13 laissant passer la lumière d'une source de lumière traversant un support translucide derrière le motif pour réaliser des lignes claires de sorte que les lignes claires du motif se reflète sur l'œil ou les yeux d'un patient.

Selon l'exemple le motif 11 de la mire 10 comporte douze lignes opaques 12 sans compter les bordures supérieure et inférieure de la mire. Ces lignes opaques sont séparées par des lignes transparentes 13 et centrées autour d'une ligne translucide médiane. La mire peut être montée sur un cadre plastique pour la rendre plus aisée à manipuler.

Par convention, on appellera axe horizontal un axe parallèle à un axe passant par les yeux du patient et axe vertical l'axe perpendiculaire à cet axe et dans l'exemple illustré, les lignes du motif sont horizontales.

Comme représenté en figure 2, la mire 10 sur son support plastique 10b est positionnée sur un support translucide 10a lui-même percé de trous de passage des objectifs caméras et le dispositif utilise une source de lumière diffuse 23 derrière le support du motif pour illuminer le motif. La réflexion du motif est observée par une ou deux caméras numériques et, pour observer les deux yeux, deux caméras numériques 21, 22 sont prévues.

La source de lumière diffuse 23 peut être réalisée au moyen d'une boîte ou sphère d'intégration ou de manière similaire à un rétroéclairage d'écran LCD par exemple.

La mire est pourvue de deux zones évidées 14 centrées dans des cadres opaques 15 sur une ligne médiane horizontale. Les zones évidées sont distantes d'une distance correspondant à un écart oculaire moyen comme représenté en figure 1. De retour à la figure 2, les caméras sont positionnées derrière les zones évidées et se retrouvent en face des yeux 101 du patient 100. Les objectifs des caméras filment ou photographient les yeux du patient au travers des zones évidées. Les zones évidées peuvent être remplacées par des zones de substrat transparentes de la mire si les propriétés optiques du substrat de la mire et son niveau de propreté sont compatibles avec la formation d'image (transparent et non diffusant).

Les caméras sont par exemple des caméras de type CMOS à capteur 1/4". Selon l'exemple non limitatif représenté, les zones transparentes sont des trous circulaires d'un diamètre adapté aux objectifs des caméras. Pour des caméras avec des optiques de focale de l'ordre de 4mm, des trous de l'ordre de 14 mm sont réalisés et les cadres opaques sont des carrés opaques de l'ordre de 16mm x 16mm. Ces cadres ont pour but de précisément terminer les lignes en amont des trous recevant les objectifs des caméras.

Dans l'exemple de réalisation, la ou les caméras fournissent des images avec une définition de 1920x1080 qui suffit pour une analyse du film oculaire sans accroître la charge de calcul du système.

Les signaux vidéo ou des caméras sont envoyés vers un dispositif de traitement informatisé 30 ou système de calcul, interne ou externe au dispositif de mesure et, pour éviter de dupliquer ce dispositif de traitement, les signaux vidéos des deux caméras passent par un multiplexeur, sur une carte électronique 24 du dispositif, le multiplexeur permettant d'envoyer l'un ou l'autre des canaux vidéo au choix vers le dispositif de traitement 30.

Les caméras filment ou photographient l'image des lignes du motif réfléchie dans la cornée du patient. Comme la cornée en première approche peut être considérée comme un dioptre sphérique, elle présente une importante courbure de champ et l'image présente une importante distorsion. Afin d'au moins partiellement compenser la distorsion et de conserver sur les images capturées des lignes dont la largeur varie peu depuis l'axe médian du motif vers le bord de l'image, le motif de la mire comporte des lignes avec une période croissante depuis l'axe central du motif vers les bords du support parallèles aux lignes. La largeur des lignes est de l'ordre de 2 à 4 mm selon la résolution recherchée. Par exemple, une ligne blanche centrale peut avoir une hauteur d'environ 2,8 mm, les lignes noires adjacentes une hauteur de 2,2 mm alors que les dernières lignes blanches ont une hauteur de 3,8 mm et les lignes noires précédant ces lignes blanches ont une hauteur de 2,8 mm, la progression étant optimisée pour compenser la courbure d'un œil standard. Dans le procédé décrit ce sont les lignes claires qui sont exploitées pour rechercher les variations de largeur ou déformations des lignes mais les lignes sombres peuvent aussi être utilisées. Le nombre et la largeur des lignes claires peut être différent des exemples donnés mais est choisi pour obtenir une définition suffisante pour une détection significative des zones de déformation du film en fonction de la résolution de la ou des caméras.

Dans le principe de la présente demande, les lignes blanches sont reflétées par le dioptre cornéen et ressortent alors qu'elles sont rétrodiffusées dans la conjonctive bulbaire de l'œil et l'iris où l'alternance lignes sombres lignes claires réalise plutôt un fond continu plus ou moins lumineux dépendant du ratio entre les surfaces transparentes de la mire et sa surface totale.

En figure 2, la mire vue de dessus est courbée autour d'un axe vertical pour former une portion de cylindre et suivre la courbure de la tête du patient 100 de sorte que l'image de la mire couvre une grande partie de la cornée.

Les raisons en sont :
a. La conjugaison optique :
   La caméra voit le reflet de la mire par la cornée considérée en première approche comme un miroir sphérique de rayon 8 mm environ, soit 4 mm de focale. Compte tenu de cette courte focale, il faut que la mire (l'objet dans la conjugaison optique) soit grande pour que la taille de l'image par la cornée soit suffisamment grande, de taille comparable au diamètre extérieur de l'iris. C'est ce qui détermine la taille du masque.
b. La photométrie :
   Pour que la mire soit visible, il faut que des rayons lumineux issus des bords extrêmes de la mire entrent dans la pupille de l'objectif. La cornée étant un miroir de forte courbure, il faut qu'en bord de champ les rayons lumineux arrivant sur la cornée soient rasant.

C'est ce qui justifie la courbure de la mire.

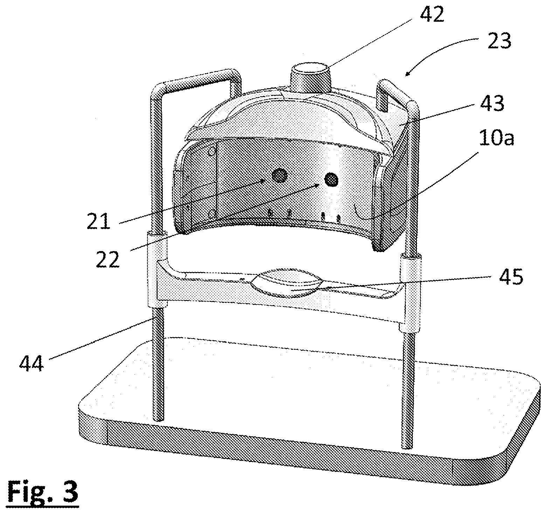

Selon la figure 3 le dispositif de mesure est monté sur un bâti ophtalmologique avec appui menton et front comportant des montants 44, 46, un carter 43 qui intègre les caméras et qui reçoit la mire 10 sur une face courbe frontale devant laquelle va se placer le patient, menton en appui sur un support 45. Sur un tel bâti, les yeux du patient sont à une distance de la mire d'environ 50 mm pour une focale de caméra de 4mm. Le dispositif de mesure peut aussi être intégré à un casque porté par le patient.

La focale et la distance sont choisies pour permettre de de voir tout l'œil compte tenu des variations de positionnement de l'œil par rapport à la caméra (écart interoculaire différent d'un individu à l'autre). Ensuite on choisit une fenêtre d'intérêt centrée sur la pupille du patient, par action de l'opérateur qui pointe le centre de la pupille sur l'image.

De sorte que les lignes de la mire soient nettes sur l'image, la mise au point se règle avec une molette 42.

Le procédé de mesure de la présente demande a pour objet de détecter et de mesurer les étendues et zones d'instabilité du film lacrymal qui se traduisent par des déformations des lignes réfléchies. Il comporte une répétition de prises de vues de l'un ou des yeux du patient selon une fréquence de répétition de l'ordre de 0.1 à 0,5 secondes et en pratique de 0,3 secondes après un clignement des yeux ou de l'œil.

Le procédé est décrit principalement dans le cadre d'une mire à lignes horizontales c'est-à-dire selon un axe passant par les deux pupilles du patient mais est adaptable notamment moyennant une rotation de 90° des moyens de traitement de séries de pixels et du filtre passe bande anisotrope décrit ci-après. Par ailleurs le procédé décrit dans le cadre de la détection des lignes claires peut s'appliquer à une détection des lignes sombres.

Utiliser des lignes horizontales ou verticales plutôt qu'inclinées est intéressant du fait que les traitements d'images le long de lignes ou de colonnes de pixels sont les plus simples à réaliser.

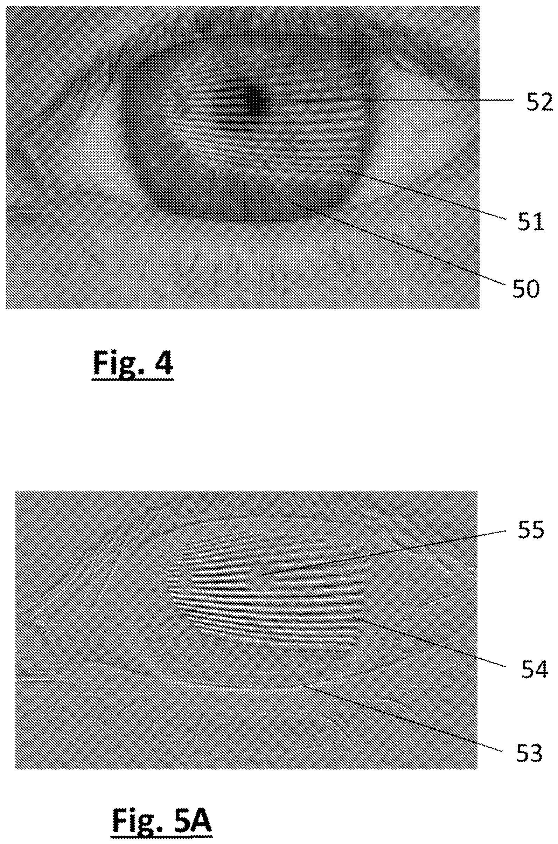

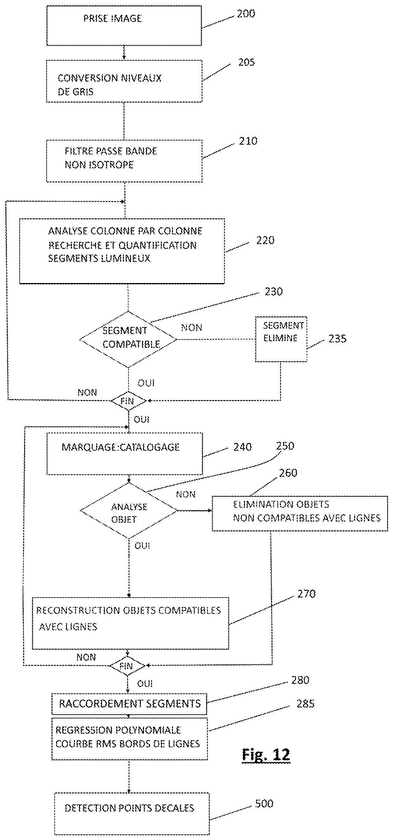

Les étapes de mesure dans le système de calcul 30 comprennent des étapes de traitement d'image qui, partant d'une capture de l'image originale de l'œil du patient comprennent;
- une conversion de l'image en niveaux de gris selon l'étape 205 de la figure 12 et dont un exemple de résultat est représenté en figure 4 où l'on distingue l'image 51 du motif sur l'iris 50 avec l'image du cadre 52 entourant l'objectif de la caméra. Sur cette image et l'image d'origine en couleurs, la forme des lignes claires réfléchies comporte des défauts locaux (notamment des bords de lignes irréguliers qui révèlent déjà des déformations du film lacrymal) ;
- une application d'un filtre passe bande anisotrope, étape 210 de la figure 12, selon une direction perpendiculaire à la direction des lignes reflétées. Cette direction est une direction verticale selon les colonnes de pixels de l'image dans le cas d'une mire à lignes horizontales ou d'une mire à lignes verticales avec l'image tournée de 90° pour obtenir des lignes claires orientées dans une direction horizontale. Le filtre est configuré pour garder les transitions franches entre les niveaux de gris et supprimer ou atténuer les modulations de basse fréquence spatiale et de fréquence spatiale plus élevée dans la direction perpendiculaire. L'image en sortie de filtre est représentée à la figure 5A. Ce filtre a notamment pour utilité de s'affranchir des phénomènes de vignettage et des défauts d'homogénéité de l'éclairage. Cette transformation accentue les lignes de la paupière 53, les lignes claires 54 du motif de la mire et conserve l'image du cadre 55.

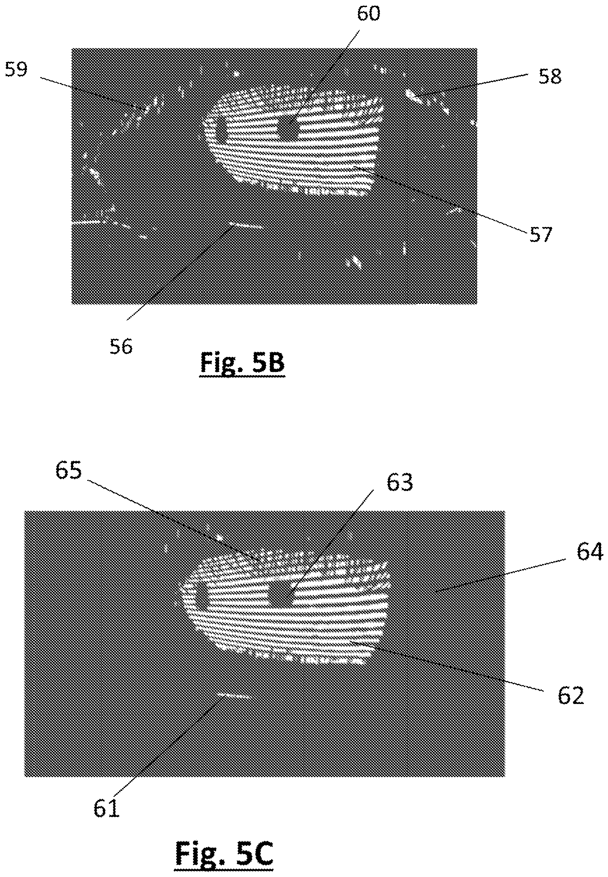

Ensuite, toujours dans le cas de lignes claires orientées dans une direction horizontale, le procédé comporte une analyse de l'image 220 par colonnes de pixels selon la figure 12 qui va rechercher des segments lumineux verticaux. L'image résultante comporte alors des lignes 56, 57, 58, 59 comme représenté en figure 5B. Une fois cette analyse faite, il est procédé à une qualification des segments lumineux par leur taille, étapes 230, 235. Ceci permet d'éliminer les segments trop grands ou trop courts qui ne correspondent clairement pas à des segments de lignes du motif, par exemple les segments faisant partie du contour des paupières. A l'issue de cette étape, la figure 5C représente l'image où subsistent les segments de colonnes constituant la ligne isolée 61, les lignes de l'image du motif 62, le fond 64 et le cadre 63. Il est à noter que les cils causent une fragmentation importante des lignes 65 du haut de l'image. Dans le cas d'une mire à lignes verticales, l'analyse et la qualification des segments se fait par lignes de pixels.

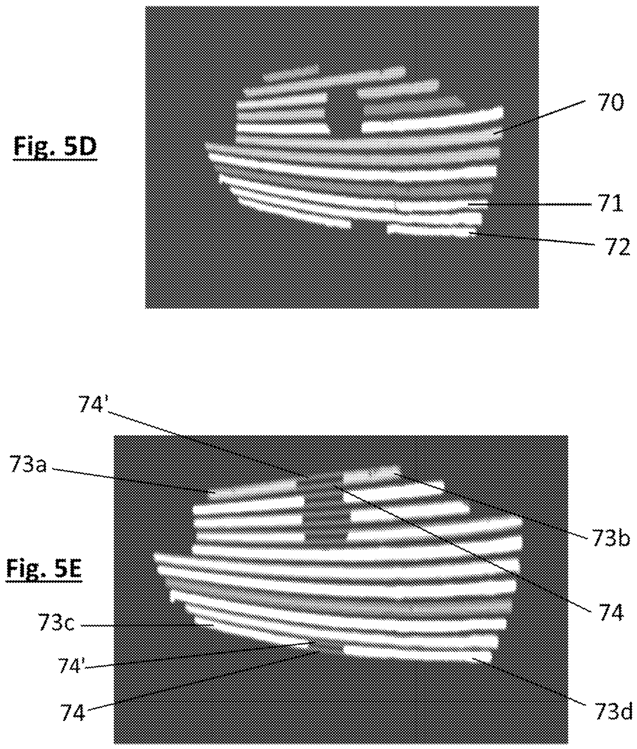

Lorsque la segmentation est terminée, le procédé de traitement comporte un algorithme de marquage/catalogage 240, 250, 260 des segments lumineux pour obtenir les objets représentatifs de branches lumineuses de lignes du motif et pour rejeter les objets lumineux n'ayant pas la forme recherchée qui sont alors considérés comme des artéfacts. Cet algorithme comporte en premier lieu un raccordement des segments de colonnes contigus pour reconstituer des branches horizontales. Le résultat de ce marquage/catalogage est représenté en figure 5D pour lequel à chaque ligne trouvée est affecté une couleur ici représentée en niveaux de gris. Ce catalogage permet de créer des lignes complètes 70 ou des branches isolées 71, 72.

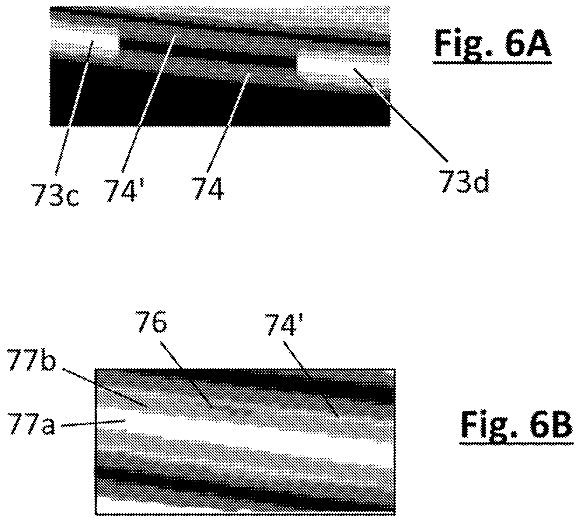

Une étape suivante est un raccordement 280 des branches de lignes lumineuses de même niveau (par exemple de largeur et d'altitude similaire) dans l'image puis une régression polynomiale 285 utilisant un polynôme d'ordre supérieur à deux en sorte de calculer une courbe RMS de la forme des bords de lignes. Cette étape est représentée en figure 5E. Dans cette figure on remarque notamment des raccordements par les courbes RMS inférieure 74 et supérieure 74' des branches 73a, 73b des lignes morcelées au niveau de l'image du cadre entourant l'objectif de la caméra et des branches 73c, 73d de la ligne inférieure. L'agrandissement de la figure 6A permet de mieux distinguer les courbes RMS 74, 74' entre les branches 73c, 73c du bas de l'image. Ensuite est réalisée une détection des points décalés 500 aux endroits où les bords des lignes comportent des points de mesure qui s'écartent de la forme donnée par le polynôme.

Contrairement à un procédé de simple recherche de ruptures de film avérées, dans le cadre de la présente invention on recherche les fluctuations d'épaisseur du film qui se traduisent par des micro-mouvements dans le film.

Le critère pris en compte est pour la détection est ici uniquement un critère de seuil (par exemple un seuil de un pixel) pour filtrer le bruit dans l'image, la totalité des écarts entre les points et le polynôme supérieurs à ce seuil étant prise en compte.

Ceci est par exemple représenté en figure 6B dans la zone 76 où le bord de ligne 77b de la ligne 77a n'atteint pas la courbe 74'.

Comme vu plus haut, le procédé peut être basé sur un traitement des lignes sombres. Grouper les paires de transition (montante et descendante dans le cas des lignes claires) permet de faire un contrôle de cohérence sur la largeur du segment obtenu et de rejeter les segments trop larges ou trop étroits pour faire partie de l'image de la mire. Une fois qu'on a déterminé les branches, la régression polynomiale est effectuée de chaque côté de la branche : un polynôme pour les transitions montantes, un polynôme pour les transitions descendantes. Le procédé de l'invention permet donc tout aussi bien chercher les segments sombres, les lignes sombres et arriver aux même régressions polynomiales et au même résultat final.

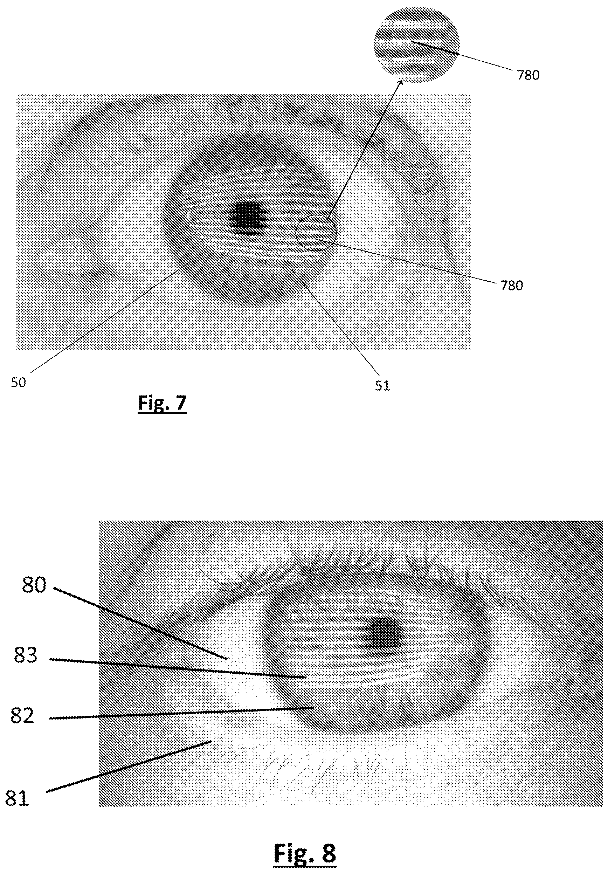

Le résultat des mesures est une cartographie des micro-mouvements dans le film lacrymal représentée à la figure 7 vue d'un œil d'un patient 6 secondes après clignement avec agrandissement pour laquelle la cartographie des zones d'épaisseur de film réduite ou nulle 780 apparaît positionnée sur les lignes 51 dans l'image initiale couleur de l'œil reprise ici en niveaux de gris.

Les micro-mouvements correspondent à des déformations ou affaissements locaux évolutifs du film par exemple en forme de cuvettes pouvant aller jusqu'à la rupture du film qui se traduisent par des déformations des lignes par modification locale de la courbure du dioptre. L'évolution de ces déformations lorsque l'on observe l'œil avec grossissement apparaît sous la forme de micro-mouvements ou ondulations.

Comme vu plus haut, les images sont capturées environ toutes les 0,3 secondes. Une origine des temps est définie comme un clignement de paupière et en répétant la mesure pour chaque image sur une période de temps permet la construction d'une carte des défauts du film oculaire en fonction du temps.

Un problème à considérer est que le regard du patient peut changer de direction pendant la période d'acquisition d'images.

Comme la caméra observe les réflexions du motif sur la cornée qui se comporte en première approximation comme un dioptre sphérique, la position de l'image du motif reste quasiment invariante dans l'image délivrée par la caméra alors que la position de l'iris change si le patient tourne les yeux. De ce fait un point donné de l'image de la mire n'est pas lié à un point fixe de la cornée, mais au contraire à un point dépendant de la direction du regard. Ceci implique que la mesure doit être référencée par rapport à la position de l'œil observé et non par rapport à l'image du motif.

Pour ce faire il faut suivre la position de l'œil dans chaque image. Il a été préféré de suivre le contour extérieur de l'iris de l'œil du fait qu'un contraste important existe avec la conjonctive bulbaire, qui est de couleur claire et sur laquelle on n'a pas de réflexion de la mire alors que la pupille est plus délicate à suivre du fait du reflet de la mire qui complique l'analyse de l'image.

Le procédé qui suit peut être mis en œuvre dans le cadre de la présente demande ou indépendamment pour d'autres mesures sur les yeux. Ce procédé est par ailleurs indépendant de l'orientation des lignes de la mire.

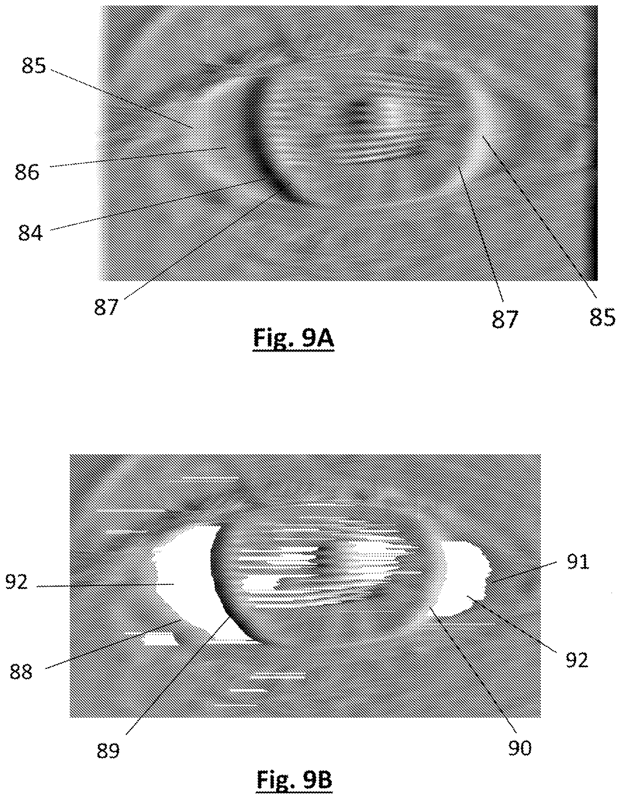

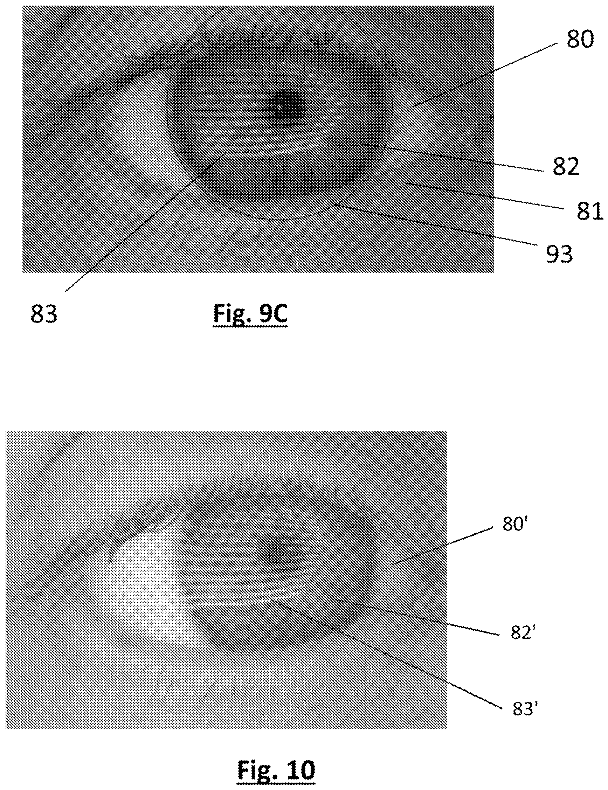

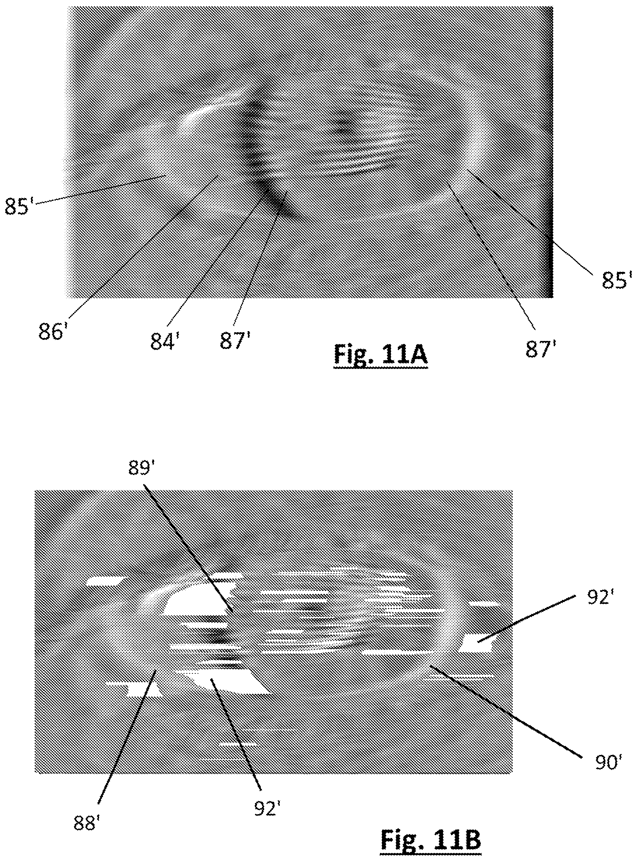

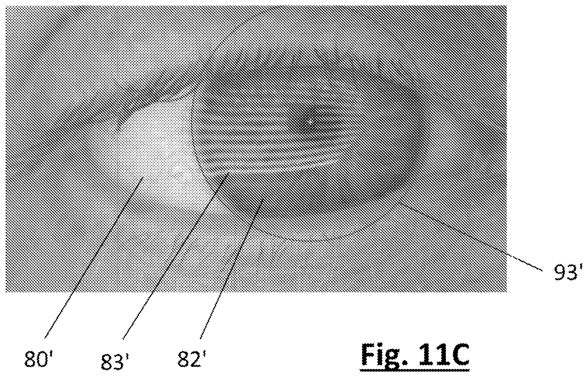

Les figures 8, 9A, 9B et 9C correspondent aux traitements d'image sur un œil d'un patient regardant la caméra et les figures 10, 11A, 11B et 11C correspondent aux traitements d'image sur un œil d'un patient dont le regard s'écarte de la caméra.

Selon la figure 8, l'œil 80 regarde droit devant, l'image du motif 83 est centrée par rapport à l'iris 82 lui-même centré par rapport à la paupière 81.

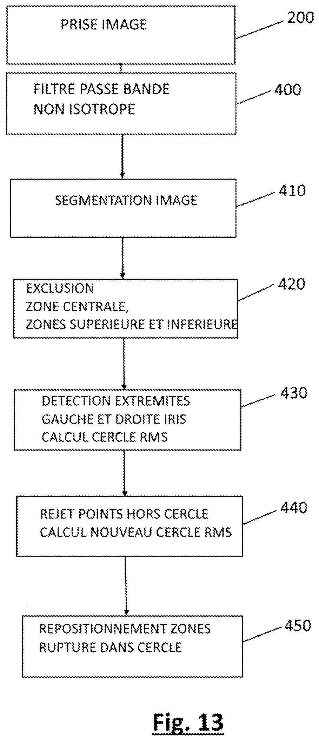

Le procédé de traitement des images pour retrouver la position de l'iris est schématisé à la figure 13. Il comporte une première transformation de l'image par l'application d'un filtre passe bande anisotrope 400 appliqué horizontalement pour détecter les transitions lumineuses selon un axe horizontal. Dans cette opération on recherche à distinguer les transitions descendantes (clair vers sombre) et les transitions montantes (sombre vers clair) et pour la compréhension, les transitions clair vers sombre (transition descendante) sont traduite par le croissant sombre 84 de la représentation en niveaux de gris de la figure 9A et les transitions sombre vers clair (transition montante) sont traduites par le croissant clair 85 de la figure 9A. Les parties de l'image sans transitions significatives deviennent gris moyen, comme le croissant 86 par exemple, et le contour de l'iris se matérialise par une portion d'anneau 87 qui se retrouve sur le côté gauche à côté d'une transition clair vers sombre et de l'autre côté de l'œil à côté d'une transition sombre vers clair.

De retour à la figure 13, une deuxième opération consiste en une segmentation de l'image 410 pour rechercher les paires de transitions montante et descendante, 84, 85 en figure 9A par exemple, qui forment des frontières des zones lumineuses dans l'image, notamment autour de la conjonctive bulbaire. Ces paires de transitions matérialisées par les limites 88, 89 et 90, 91 sur la figure 9B encadrent des zones 92 définissant potentiellement la conjonctive bulbaire.

A partir de cette transformation le procédé comporte selon la figure 13 un filtrage de l'image 420 qui supprime la zone centrale comportant le motif et les zones supérieure et inférieure de l'image. Sur les parties restantes est réalisé un calcul d'un cercle RMS du tour de l'iris à partir des extrémités droites des segments à gauche de l'image et des extrémités gauches des segments à droite de l'image 430. Pour ce calcul, les points trop éloignés du cercle RMS qui correspondent à des imperfections notamment causées par les cils ou la paupière sont rejetés à l'étape 440 et, pour les points restants, un nouveau cercle RMS est calculé pour suivre le contour de l'iris, ce cercle 93 est représenté en figure 9C sur l'image d'origine de l'œil.

La figure 10 représente un œil 80' regardant de biais et dont l'iris 82' est décalé par rapport au motif 83'. Pour cette position de l'œil, les transitions 84', 85' autour de zones sombres 86', 87' correspondant à des couleurs homogènes sont décalées latéralement dans la figure 11A alors que dans la figure 11B on constate que les arcs de cercles 89' et 90' correspondant au bord de l'iris restent détectables. L'application du procédé de suivi conduit là aussi à recréer le cercle RMS 93' qui va être repositionné sur l'image d'origine comme représenté en figure 11C. Les zones de déformation détectées sont alors relocalisées selon l'étape 460 selon la position du cercle définissant le contour de l'iris. Ceci permet d'ancrer les déformations du film lacrymal au contour de l'œil plutôt qu'à l'image.

Cette séquence est réalisée pour chaque image préférablement après l'analyse du motif en lignes décrit plus haut.

Comme dit plus haut ce procédé est ici appliqué au repositionnement des déformations du film mais il peut aussi être utilisé pour d'autres types de détections et méthodes utilisant un suivi de la position d'un œil.

Selon un aspect de la demande, le dispositif peut comporter un déclencheur manuel qui arme le dispositif, le déclenchement de la séquence de prises de vues étant alors réalisé sur l'occurrence d'un évènement tel qu'une série de deux clignements de paupières du patient. Pour ce faire, le système comporte un procédé de reconnaissance de clignement de paupières qui permet de démarrer la séquence de mesure automatiquement. De même le système peut arrêter la séquence de mesures automatiquement sur détection d'un clignement de paupières ultérieur ou arrêter la séquence automatiquement après une temporisation, par exemple 15 secondes.

La séquence de prise de vues peut comporter de 30 à 50 images par exemple et dans le cas d'une séquence de prise de vues de 15 secondes avec prise de vues toutes les 0,3 secondes la séquence comporte 45 images. L'analyse des images peut se faire après la séquence de prise de vues et du fait de la solution choisie de travailler sur un motif fait de lignes, le temps de traitement reste réduit, par exemple de 15 secondes avec un ordinateur standard.

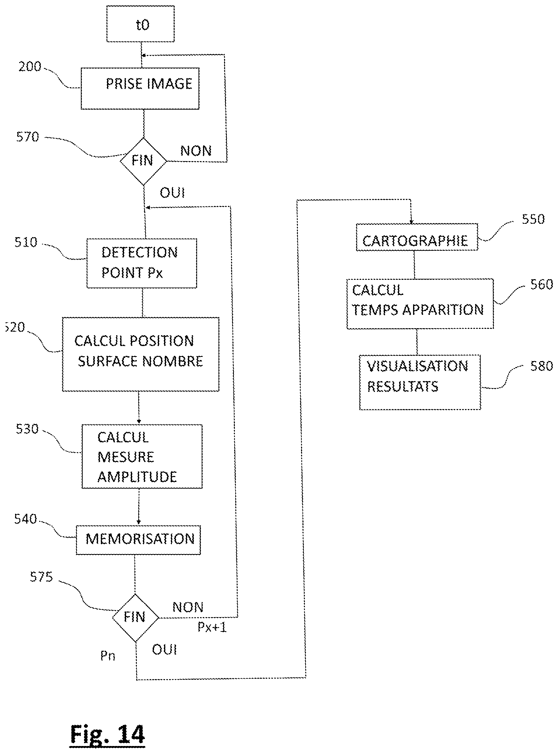

Selon la figure 14, le procédé comporte, à partir d'une origine des temps t0 constituée par un clignement de paupière, une succession de prises d'images 200 sur une séquence qui va durer une quinzaine de secondes et prenant des images tous les 0,1 à 0,5 secondes et préférablement toutes les 0,3 secondes pour avoir un bon compromis entre la détection des variations du film oculaire et la quantité de données à traiter.

La succession de prises d'images se termine avec le premier arrivé de l'un des évènements : fin d'une temporisation, par exemple 15 secondes ce qui donne 45 images avec une image toutes les 0.3 secondes ou détection d'un clignement de paupière suivant 570.

Entre les prises d'images ou en différé, le procédé comporte une succession de détections de zones de micro mouvements dans les images 510 et de calculs et mémorisation de données de positions, de surface et de nombre de zones de micro-mouvements 520 du film lacrymal dans chaque image au moyen des moyens de traitement et d'analyse d'images selon par exemple la méthode de la figure 12 et notamment la régression polynomiale qui va permettre de déterminer des bords de lignes estimés au moyen d'un polynôme de régression polynomiale.

Le procédé comporte ensuite le calcul 530 et la mémorisation 540, d'une mesure de l'amplitude des zones de micro-mouvements en fonction du temps. Cette mesure donne un aperçu des réductions d'épaisseur du film sur toute l'étendue des lignes visualisées.

Pour recaler les positions des lignes entre les images, le procédé va comporter le suivi de l'œil du patient objet de la figure 13.

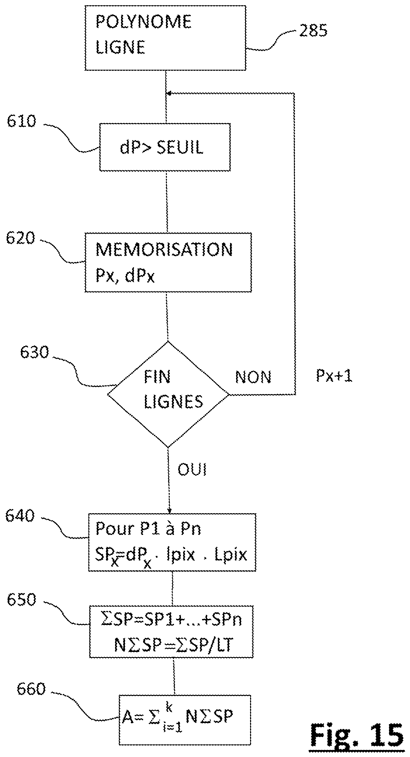

Dans une étape suivante selon la figure 15, le procédé va permettre de déterminer une population de points représentatifs des zones de micro-mouvements observées sur l'image des lignes.

Ceci est fait par un calcul 610, le long des lignes, de la valeur absolue de la distance au polynôme selon l'axe perpendiculaire à la direction générale des lignes des pixels P1 à Pn des bords de lignes de l'image qui sont écartés du polynôme d'une distance dP en pixels supérieure à un seuil 620. Ces points représentatifs P1 à Pn et les distances dP1 à dPn sont alors mémorisés. Dans le cas de lignes horizontales, la distance est verticale et dans le cas de lignes verticales, la distance est horizontale.

Pour déterminer une étendue de zones où le film est déformé, le procédé comporte pour chaque image, un calcul 640 pour chaque pixel P1 à Pn de la surface équivalente de la déformation au niveau du bord de ligne Sp = dP x largeur pixel x hauteur pixel.

Ensuite un calcul 650 de la somme ΣSp des surfaces équivalents SP1 à SPn entre les points image et leur polynôme sur toute l'image et d'une somme normalisée NΣSp desdites distances au moyen de la division de la somme ΣSp par la longueur totale LT des lignes trouvées sur l'image.

Ce nombre représente une étendue globale des déformations dans les zones des bords de lignes.

Pour obtenir une valeur reproductible entre les examens, le procédé comporte le calcul 660 d'un score global constitué par la somme A des sommes normalisées NΣSp sur une série d'images à partir de l'origine des temps t0 jusqu'à un instant t donné.

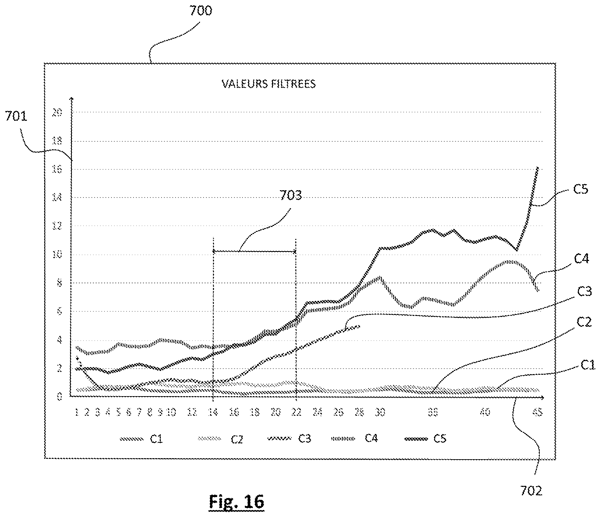

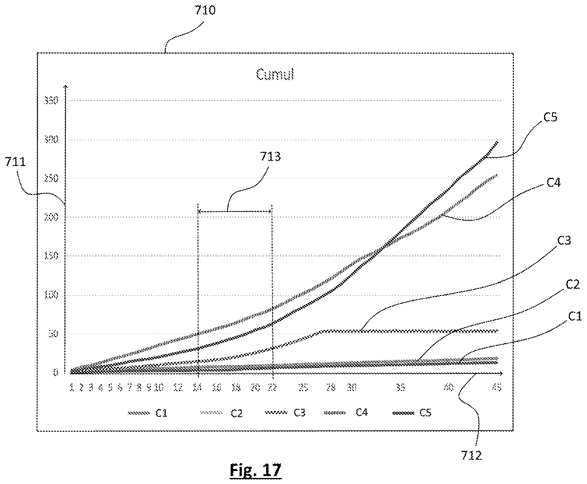

Selon les exemples des courbes des figures 16 et 17 on constate qu'une somme sur une durée de l'ordre de 6 secondes soit 18 images lorsque les images sont prises toutes les 0,3 secondes permet de distinguer les différences entre patients.

Le procédé peut en outre comporter la génération 550 d'une cartographie des localisations des micro-mouvements image par image ce qui permet de distinguer les zones d'instabilité plus marquées du film oculaire et d'observer la variation des déformations du film dans le temps.

Il est aussi possible de calculer et de mémoriser le temps d'apparition des points de micro-mouvements du film lacrymal et/ou de la cinétique d'apparition de points de micro-mouvements du film lacrymal 560 ce qui peut être utile pour comparer les résultats au cours d'un traitement.

En outre toute ou partie des données calculées peuvent être mémorisées en sorte de réaliser un suivi du patient et une comparaison desdites données sur plusieurs examens.

Lorsque la mesure est terminée, le praticien a à disposition, d'une part, une cartographie spatiale et temporelle des micro-mouvements du film lacrymal au sein de la surface cornéenne et, d'autre part, une courbe temporelle retraçant l'évolution de ces micro-mouvements du film lacrymal en fonction du temps. L'amplitude en chaque point de la courbe va révéler le niveau de stabilité à un instant donné. La pente de cette courbe temporelle, va révéler la cinétique d'apparition des ruptures du film lacrymal. Ceci permet d'affiner l'interprétation de l'examen pratiqué.

La figure 16 représente un graphique 700 avec en ordonnée 701 les somme normalisée NΣSp filtrées avec une moyenne glissante sur trois images et en abscisse 702 les images pour cinq patients C1 à C5.

Les patients C1 et C2 présentent peu micro-mouvements c'est à dire une faible instabilité due leur film oculaire en fonction du temps, le patient C3 présente une instabilité moyenne mais n'a pas tenu l'œil ouvert pendant la séquence complète alors que les patients C4 et C5 présentent une grande instabilité représentative de sécheresse oculaire.

La figure 17 représente un graphique 710 avec en ordonnée 711 la valeur cumulée A entre les images successives et en abscisse 702 les images. Ce graphique amplifie la visibilité de la croissance des zones de micro-mouvements pour les patients C4 et C5 atteints de sécheresse oculaire tandis que les patients C1 et C2 montrent une progression très réduite, le patient C3 montrant une progression moyenne jusqu'au clignement d'œil au niveau de l'image 28.

Sur les deux graphiques ont été représentées des zones temporelles 703, 713 d'environ deux secondes centrées autour de la 18^{ème} image, soit dans le cas présent 6 secondes après clignement. On constate sur les courbes que cette durée d'examen de l'ordre de 6 secondes peut déjà permettre de normaliser les résultats et de distinguer les patients sans problèmes de sécheresse oculaire de ceux qui en sont atteints sans risquer d'arriver à une durée d'examen pouvant occasionner des clignements d'œil intempestifs.

L'invention ne se limite pas aux exemples décrits ci-avant, seulement à titre d'exemple, mais elle englobe toutes les variantes comme une distribution ou une progression de la hauteur des lignes différentes que pourra envisager l'homme de l'art dans le cadre de la protection recherchée. En particulier, comme dit plus haut, les lignes du motif qui sont des lignes parallèles horizontales selon l'exemple représenté peuvent être remplacées par des lignes parallèles verticales, une rotation de l'image permettant par exemple d'appliquer les moyens de traitement d'image de détection des déformations des lignes à cette configuration sans en changer la direction.

## Revendications

1. Procédé de mesure de la stabilité d'un film lacrymal d'un patient au moyen d'un dispositif comportant :
- une plaque translucide rétroéclairée (10a) munie d'une mire (10) se positionnant devant au moins un œil (101) d'un patient (100), ladite mire étant pourvue d'un motif constitué d'une succession de lignes claires et sombres (12, 13) alternées se réfléchissant sur l'œil du patient,
- au moins une caméra photographique numérique (22, 23) reliée à un système de calcul (30) pourvu de moyens de traitement et d'analyse d'images, un objectif de la caméra pointant vers l'œil du patient afin de photographier une réflexion du motif de la mire sur l'œil du patient,
ledit procédé étant tel que
- les moyens de traitement et d'analyse d'images sont configurés pour
- détecter des déformations (76) desdites lignes claires ou sombres du motif de la mire réfléchi sur l'œil du patient et
- identifier, par une comparaison de la position de points images du bord des lignes par rapport à un bord des lignes estimé (74, 74'), les micro-mouvements du film lacrymal révélés par ces déformations,
le procédé comportant, à partir d'une origine des temps (t0) constituée par un clignement de paupière :
- une succession de prises d'images (200),
- une succession de détections de zones de micro-mouvements dans les images (510) et de calculs et mémorisation de données de positions, de surface et de nombre de zones de micro-mouvements (520) du film lacrymal dans chaque image au moyen des moyens de traitement et d'analyse d'images et
- le calcul (530) et la mémorisation (540), à partir de la succession de calculs de positions surface et nombre de zones de micro-mouvements du film lacrymal, d'une mesure d'une amplitude des zones de micro-mouvements en fonction du temps.

2. Procédé de mesure de la stabilité d'un film lacrymal selon la revendication 1, comportant une prise d'image (200) toutes les 0,1 à 0,5 secondes et préférablement toutes les 0,3 secondes.

3. Procédé de mesure de la stabilité d'un film lacrymal selon la revendication 1 ou 2, pour lequel la succession de prises d'images se termine avec le premier arrivé de l'un des évènements : fin d'une temporisation ou détection d'un clignement de paupière suivant (570).

4. Procédé de mesure de la stabilité d'un film lacrymal selon la revendication 1, 2 ou 3 comportant un suivi de l'œil ou des yeux du patient (400, 410, 420, 430, 440, 460) au moyen d'un procédé de suivi d'iris en sorte de repositionner les zones de déformation du film lacrymal détectées et mémorisées par rapport à l'œil analysé.

5. Procédé de mesure de la stabilité d'un film lacrymal selon l'une quelconque des revendications précédentes pour lequel les bords des lignes estimés sont calculés par régression polynomiale (285).

6. Procédé de mesure de la stabilité d'un film lacrymal selon la revendication 5 comportant la détermination d'une population de points représentatifs des zones de micro-mouvements observées sur l'image des lignes par calcul (610), le long des lignes, de la valeur absolue de la distance au polynôme selon l'axe perpendiculaire à la direction générale des lignes des pixels P1 à Pn des bords de lignes de l'image qui sont écartés du polynôme d'une distance dP en pixels supérieure à un seuil (620) et mémorisation (620) pour les points représentatifs P1 à Pn des distances dP1 à dPn.

7. Procédé de mesure de la stabilité d'un film lacrymal selon la revendication 6 comportant pour chaque image, sur ladite population de points représentatifs, un calcul pour chaque pixel P1 à Pn de la surface équivalente Sp = dP x largeur pixel x hauteur pixel (640) et un calcul (650) de la somme ΣSp des surfaces équivalents SP1 à SPn des points image à leur polynôme sur toute l'image et d'une somme normalisée NΣSp desdites distances au moyen de la division de la somme ΣSp par la longueur totale LT des lignes trouvées sur l'image.

8. Procédé de mesure de la stabilité d'un film lacrymal selon la revendication 7 comportant le calcul (660) d'un score global constitué par la somme A des sommes normalisées NΣSp sur une série d'images à partir de l'origine des temps t0 jusqu'à un instant t donné.

9. Procédé de mesure de la stabilité d'un film lacrymal selon l'une quelconque des revendications précédentes comportant la génération (550) d'une cartographie des localisations des micro-mouvements image par image.

10. Procédé de mesure de la stabilité d'un film lacrymal selon l'une quelconque des revendications précédentes comportant le calcul et la mémorisation (560) du temps d'apparition des points de micro-mouvement d'un film lacrymal et/ou de la cinétique d'apparition de points de micro-mouvements du film lacrymal.

11. Procédé de mesure de la stabilité d'un film lacrymal selon l'une quelconque des revendications précédentes comportant la mémorisation dans une base de données de toute ou partie des données calculées en sorte de réaliser un suivi du patient et une comparaison desdites données sur plusieurs examens.

12. Programme informatique comportant des instructions pour la mise en œuvre du procédé selon l'une quelconque des revendications précédentes lorsque ce programme est exécuté par un processeur.

13. Support d'enregistrement non transitoire lisible par un ordinateur sur lequel est enregistré un programme pour la mise en œuvre du procédé selon l'une quelconque des revendications 1 à 11 lorsque ce programme est exécuté par un processeur.

## Patentansprüche

1. Verfahren zur Messung der Stabilität eines Tränenfilms eines Patienten mittels einer Vorrichtung, umfassend:
- eine lichtdurchlässige, hinterleuchtete Platte (10a) mit einem Visier (10), das vor wenigstens einem Auge (101) eines Patienten (100) positioniert wird, wobei das Visier mit einem Muster versehen ist, das aus einer Folge von abwechselnd hellen und dunklen Linien (12, 13) besteht, die auf dem Auge des Patienten reflektiert werden,
- wenigstens eine digitale Fotokamera (22, 23), die mit einem Rechnersystem (30) verbunden ist, das mit Mitteln zur Bildverarbeitung und -analyse versehen ist, wobei ein Objektiv der Kamera auf das Auge des Patienten zeigt, um eine Reflexion des Musters des Visiers auf dem Auge des Patienten zu fotografieren,
wobei das Verfahren derart ist, dass
- die Bildverarbeitungs- und -analysemittel dazu ausgebildet sind,
- Verformungen (76) der genannten hellen oder dunklen Linien des vom Auge des Patienten reflektierten Musters des Visiers zu erfassen und
- durch einen Vergleich der Position von Bildpunkten des Randes der Linien in Bezug auf einen geschätzten Rand der Linien (74, 74') die Mikrobewegungen des Tränenfilms zu identifizieren, die durch diese Verformungen aufgedeckt werden,
wobei das Verfahren ausgehend von einem Zeitursprung (t0), der durch einen Lidschlag gebildet wird, umfasst:
- eine Folge von Bildaufnahmen (200),
- eine Folge von Erfassungen von Mikrobewegungszonen in den Bildern (510) und von Berechnungen und Speichern von Daten der Positionen, der Fläche und der Anzahl von Mikrobewegungszonen (520) des Tränenfilms in jedem Bild mit Hilfe von Mitteln zur Bildverarbeitung und -analyse und
- Berechnen (530) und Speichern (540) eines Maßes einer Amplitude der Mikrobewegungszonen als Funktion der Zeit, ausgehend von der Folge von Berechnungen von Positionen, Fläche und Anzahl von Mikrobewegungszonen des Tränenfilms.

2. Verfahren zur Messung der Stabilität eines Tränenfilms nach Anspruch 1, das eine Aufnahme eines Bildes (200) alle 0,1 bis 0,5 Sekunden und bevorzugt alle 0,3 Sekunden umfasst.

3. Verfahren zur Messung der Stabilität eines Tränenfilms nach Anspruch 1 oder 2, wobei die Folge von Bildaufnahmen mit dem ersten Eintreffen eines der Ereignisse endet: Ende eines Timings oder Erkennung eines nachfolgenden Lidschlags (570).

4. Verfahren zur Messung der Stabilität eines Tränenfilms nach Anspruch 1, 2 oder 3, wobei das Auge oder die Augen des Patienten (400, 410, 420, 430, 440, 460) mit Hilfe eines Iris-Tracking-Verfahrens verfolgt werden, um die erfassten und gespeicherten Verformungsbereiche des Tränenfilms in Bezug auf das analysierte Auge neu zu positionieren.

5. Verfahren zur Messung der Stabilität eines Tränenfilms nach einem der vorhergehenden Ansprüche, wobei die geschätzten Linienränder durch polynomiale Regression (285) berechnet werden.

6. Verfahren zur Messung der Stabilität eines Tränenfilms nach Anspruch 5, umfassend die Bestimmung einer Population von Punkten, die repräsentativ für die auf dem Bild der Linien beobachteten Mikrobewegungenszonen sind, durch Berechnung (610), entlang der Linien, des Absolutwerts des Abstands zum Polynom entlang der Achse senkrecht zur allgemeinen Richtung der Linien der Pixel P1 bis Pn der Linienränder des Bildes, die vom Polynom um einen Abstand dP in Pixeln größer als ein Schwellenwert (620) entfernt sind, und Speichern (620) der Abstände dP1 bis dPn für die repräsentativen Punkte P1 bis Pn.

7. Verfahren zur Messung der Stabilität eines Tränenfilms nach Anspruch 6, das für jedes Bild auf der genannten Population repräsentativer Punkte eine Berechnung für jedes Pixel P1 bis Pn der äquivalenten Fläche Sp = dP x Pixelbreite x Pixelhöhe (640) und eine Berechnung (650) der Summe ΣSp der äquivalenten Flächen SP1 bis SPn der Bildpunkte zu ihrem Polynom über das gesamte Bild und einer normalisierten Summe NΣSp der genannten Abstände mittels der Division der Summe ΣSp durch die Gesamtlänge LT der auf dem Bild gefundenen Linien umfasst.

8. Verfahren zur Messung der Stabilität eines Tränenfilms nach Anspruch 7, welches die Berechnung (660) eines Gesamtscores umfasst, der aus der Summe A der normalisierten Summen NΣSp über eine Reihe von Bildern ab dem Zeitursprung t0 bis zu einem gegebenen Zeitpunkt t besteht.

9. Verfahren zur Messung der Stabilität eines Tränenfilms nach einem der vorhergehenden Ansprüche, welches die Erzeugung (550) einer Kartografie der Orte der Mikrobewegungen Bild für Bild umfasst.

10. Verfahren zur Messung der Stabilität eines Tränenfilms nach einem der vorhergehenden Ansprüche, umfassend das Berechnen und Speichern (560) der Zeit des Auftretens von Mikrobewegungspunkten eines Tränenfilms und/oder der Kinetik des Auftretens von Mikrobewegungspunkten des Tränenfilms.

11. Verfahren zur Messung der Stabilität eines Tränenfilms nach einem der vorhergehenden Ansprüche, welches das Speichern aller oder eines Teils der berechneten Daten in einer Datenbank umfasst, so dass eine Nachverfolgung des Patienten und ein Vergleich dieser Daten über mehrere Untersuchungen hinweg durchgeführt werden kann.

12. Computerprogramm mit Anweisungen zur Durchführung des Verfahrens nach einem der vorhergehenden Ansprüche, wenn dieses Programm von einem Prozessor ausgeführt wird.

13. Computerlesbares, nicht transitorisches Aufzeichnungsmedium, auf dem ein Programm zur Durchführung des Verfahrens nach einem der Ansprüche 1 bis 11 aufgezeichnet ist, wenn dieses Programm von einem Prozessor ausgeführt wird.

## Claims

1. A method for measuring the stability of a tear film of a patient by means of a device comprising:
- a backlit translucent plate (10a) equipped with a test chart (10) that is positioned in front of at least one eye (101) of a patient (100), the test chart being provided with a pattern consisting of a succession of alternating light and dark lines (12, 13) that reflect from the eye of the patient,
- at least one digital photographic camera (22, 23) connected to a computing system (30) provided with means for processing and analyzing images, an objective of the camera pointing toward the patient's eye in order to photograph a reflection of the pattern of the test chart from the patient's eye,
said method being such that,
- the means for processing and analyzing images are configured to
- detect deformations (76) of said light or dark lines of the pattern of the test chart reflected from the eye of the patient and
- identify, via a comparison of the position of image points on the edge of the lines with respect to an estimated line edge (74, 74') of the lines, tear-film micro-movements revealed by these deformations,
the method comprises, from a start time (t0) at which an eyelid blinks:
- successively capturing (200) images,
- successively detecting (510) regions of micro-movement in the images and successively computing and storing data on the positions, area and number of regions of micro-movements (520) of the tear film in each image by means of the means for processing and analyzing images, and
- computing (530) and storing (540), on the basis of the successively computed positions, area and number of regions of micro-movement of the tear film, a measurement of the amplitude of the regions of micro-movement as a function of time.

2. The method for measuring the stability of a tear film as claimed in claim 1, comprising capturing (200) an image every 0.1 to 0.5 seconds and preferably every 0.3 seconds.

3. The method for measuring the stability of a tear film as claimed in claim 1 or 2, wherein the step of successively capturing images ends (570) with the first occurrence of one of the following events: end of a time delay or detection of a next eyelid blink.

4. The method for measuring the stability of a tear film as claimed in claim 1, 2 or 3, comprising tracking (400, 410, 420, 430, 440, 460) the patient's eye or eyes by means of an iris-tracking method, so as to reposition the regions of deformation of the tear film that are detected and stored with respect to the analyzed eye.

5. The method for measuring the stability of a tear film as claimed in any one of the preceding claims, wherein the estimated line edges are computed by polynomial regression (285).

6. The method for measuring the stability of a tear film as claimed in claim 5, comprising determining a population of points representative of the regions of micro-movement observed in the image of the lines by computing (610), along the lines, the absolute value of the distance to the polynomial along the axis perpendicular to the general direction of the lines of pixels P1 to Pn of line edges of the image that are separated from the polynomial by a distance dP in pixels larger than a threshold (620), and storing (620), for the representative points P1 to Pn, the distances dP1 to dPn.

7. The method for measuring the stability of a tear film as claimed in claim 6, comprising, for each image, for said population of representative points, computing (640), for each pixel P1 to Pn, the equivalent area Sp = dP x pixel width x pixel height, and computing (650) the sum ΣSp of the equivalent areas SP1 to SPn from the image points to their polynomial over the entire image and a normalized sum NΣSp of said distances by means of division of the sum ΣSp by the total length LT of the lines found in the image.

8. The method for measuring the stability of a tear film as claimed in claim 7, comprising computing (660) an overall score consisting of the sum A of the normalized sums NΣSp over a series of images from the start time t0 to a given time t.

9. The method for measuring the stability of a tear film as claimed in any one of the preceding claims, comprising generating (550) a map of the locations of the micro-movements image by image.

10. The method for measuring the stability of a tear film as claimed in any one of the preceding claims, comprising computing and storing (560) the time of appearance of the points of micro-movement of a tear film and/or the rate of appearance of points of micro-movement of the tear film.

11. The method for measuring the stability of a tear film as claimed in any one of the preceding claims, comprising storing, in a database, all or some of the computed data with a view to monitoring the patient and comparing said data over a plurality of examinations.

12. A computer program comprising instructions for implementing the method as claimed in any one of the preceding claims when this program is executed by a processor.

13. A computer-readable non-volatile storage medium on which is stored a program for implementing the method as claimed in any one of claims 1 to 11 when this program is executed by a processor.
